(19) Europäisches Patentamt
European Patent Office
Office européen des brevets

(11) Veröffentlichungsnummer : **0 240 703**
**B1**

(12) # EUROPÄISCHE PATENTSCHRIFT

(45) Veröffentlichungstag der Patentschrift : **05.04.89**

(51) Int. Cl.⁴ : **B 01 J 38/52**, C 07 C 43/04, C 07 C 69/14

(21) Anmeldenummer : **87102883.3**

(22) Anmeldetag : **28.02.87**

(54) **Verfahren zur Reinigung und Rückgewinnung der bei Carbonylierung von Methylacetat und/oder Dimethylether anfallenden verunreinigten Katalysatorlösung.**

(30) Priorität : **29.03.86 DE 3610603**

(43) Veröffentlichungstag der Anmeldung : **14.10.87 Patentblatt 87/42**

(45) Bekanntmachung des Hinweises auf die Patenterteilung : **05.04.89 Patentblatt 89/14**

(84) Benannte Vertragsstaaten : **AT BE CH DE FR GB IT LI NL**

(56) Entgegenhaltungen :
**EP-A- 0 128 439**
**EP-A- 0 200 023**
**WO-A-82 /018 29**
**US-A- 4 364 907**

(73) Patentinhaber : **HOECHST AKTIENGESELLSCHAFT**
**Postfach 80 03 20**
**D-6230 Frankfurt am Main 80 (DE)**

(72) Erfinder : **Erpenbach, Heinz, Dr.**
**Oberbuschweg 22**
**D-5000 Köln (DE)**
Erfinder : **Gehrmann, Klaus, Dr.**
**Geschwister-Scholl-Strasse 32**
**D-5042 Erftstadt (DE)**
Erfinder : **Lork, Winfried**
**Siegfried-von-Westerburg-Strasse 14**
**D-5042 Erftstadt (DE)**
Erfinder : **Prinz, Peter**
**Von-Geyr-Ring 104**
**D-5030 Hürth (DE)**

**EP 0 240 703 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Reinigung und Rückgewinnung der bei der Carbonylierung von Methylacetat und/oder Dimethylether anfallenden verunreinigten Katalysatorlösung, welche Carbonylkomplexe des Rhodiums, quaternäre heterocyclische aromatische Stickstoffverbindungen oder quaternäre Organophosphorverbindungen als organische Promotoren und/oder Alkalimetallsalze und ggf. Verbindungen carbonylbildender Nichtedelmetalle als anorganische Promotoren, undestillierbare organische Verunreinigungen sowie Essigsäure, Essigsäureanhydrid und Ethylidendiacetat enthält.

Zur Durchführung von Hydroformylierungs- und Carbonylierungsverfahren wird als Edelmetallkatalysator Rhodium in Form vielfältiger komplexer Verbindungen eingesetzt. Da Rhodium aufgrund seiner geringen Verfügbarkeit ein sehr teures Edelmetall ist, wird über seine Rückgewinnung bzw. die Reinigung von Rhodiumkomplexen aus mit Rückständen verunreinigten Katalysatorsystemen bzw. Destillationssümpfen der oben angeführten Reaktionen mehrfach in der Literatur berichtet.

Den Unterschied im Löseverhalten des komplexen Rh-Katalysators und der anfallenden Teere belegen die Verfahren der EP-A-0 073 922 und 0 073 342. Beide Verfahren gehen bei der Reinigung und Rückgewinnung der bei der Carbonylierung von Methylacetat und/oder Dimethylether anfallenden verunreinigten Katalysatorlösung von einem Rhodium-Phosphonium- oder Rhodium-Ammonium-Carbonylkomplex als Katalysator aus. In beiden Fällen werden zunächst die flüchtigen Bestandteile aus der verunreinigten Katalysatorlösung entfernt. Gemäß EP-A-0 073 922 erfolgt die Entfernung der organischen Verunreinigungen aus dem verbleibenden Rückstand durch Extraktion mit aliphatischen Ethern. Gemäß EP-A-0 073 342 wird der Rückstand nach Abtrennen der flüchtigen Bestandteile zunächst mit Hilfe von Wasser in den wasserlöslichen organischen Promotor und in ein wasserunlösliches Gemisch aus Rhodiumcarbonylkomplex und organischen Verunreinigungen aufgetrennt. Aus dem wasserunlöslichen Rückstand werden sodann unter Verwendung aliphatischer Ether die organischen Verunreinigungen herausgelöst. In beiden Fällen bleibt der Edelmetall-Carbonylkomplex erhalten und gelangt wieder in den Reaktionskreislauf, während die gebildeten Rückstände mit Ausbeuten von mehr als 90 % jeweils aus der Etherphase isoliert werden. Die Rhodium- bzw. Edelmetallausbeute erreicht Werte zwischen 98,8 und 99,6 %.

Die EP-A-0 128 439 beschreibt ein Verfahren, das es ermöglicht, den bei der Carbonylierung von Methylacetat und/oder Dimethylether eingesetzten und im Laufe des Prozesses verunreinigten Katalysatorkomplex so aufzuarbeiten, daß unter Extraktion der undestillierbaren organischen Verunreinigungen mit Ethylenglykoldialkylethern der Formel $R(OC_2H_4)_n$—OR (n = 1-4) das Edelmetall der Gruppe VIII in elementarer Form ausfällt und mit Ausbeuten > 99,9 %, bezogen auf eingesetztes Edelmetall, zurückgewonnen wird. Hier ist vor Wiedereinsatz des Rhodiums in die Reaktion eine Überführung des elementaren Rhodiums in einen löslichen Rh-Carbonylkomplex erforderlich.

Der hohe Preis des Rhodiums gestattet nur seine verlustfreie Verwendung als Katalysator für großtechnische Verfahren. Dies gilt auch für die Reinigung und Rückgewinnung eines eingesetzten verunreinigten Rhodiumkatalysators und macht daher einen quantitativen Kreislauf unerläßlich. Dies erfordert eine ohne Zwischenstufen verlaufende, möglichst einfache Abtrennung des im Verfahren gebildeten organischen Rückstandes aus der Katalysatorlösung ohne jeden Rh-Verlust und den direkten Wiedereinsatz des gereinigten Katalysatorsystems in die Raktion.

Die Verfahren der EP-A-0 073 922, 0 073 342 und 0 128 439 erfüllen diese Forderungen bereits weitgehend, jedoch nicht quantitativ. Während in den ersten beiden Fällen bei Rh-Ausbeuten von höchstens 99,6 % der Rh-Carbonylkomplex unzerstört bleibt, gelingt es in der zuletzt zitierten Anmeldung, die Rh-Ausbeute nach der Reinigung noch auf Werte > 99,9 % zu steigern. Doch hierbei fällt das Rhodium, wie erwähnt, in metallischer Form an und muß durch zusätzliche Maßnahmen in die Komplexform überführt werden.

Die Summe der Forderungen wird nun durch die vorliegende Erfindung erfüllt, die ein Verfahren beschreibt, das es überraschenderweise ermöglicht, die bei der Carbonylierung von Methylacetat und/oder Dimethylether eingesetzte und im Laufe des Prozesses verunreinigte Katalysatorlösung durch Extraktion und einfache Destillation so aufzuarbeiten, daß eine Abtrennung der undestillierbaren organischen Verunreinigungen aus der Katalysatorlösung ohne Rhodiumverlust und ohne Zerstörung des Rh-Carbonylkomplexes sowie des Promotors erfolgen kann. Rh-Carbonylkomplex und Promotorsalz können so ohne aufwendige Rekombinationsmaßnahmen dem Carbonylierungsprozeß wieder zugeführt werden. Gleichzeitig wird durch den Kreislaufbetrieb der zur Aufarbeitung eingesetzten Extraktionsmittel eine zusätzliche Umweltbelastung durch Abfallstoffe vermieden. Nur die im Prozeß gebildeten undestillierbaren organischen Verunreinigungen werden ausgeschleust und können entsprechend dem Stand der Technik beseitigt werden. Der Extraktionsprozeß des Verfahrens der Erfindung wird in Form einer Flüssig-Flüssig-Extraktion durchgeführt. Da es beim Verfahren der Erfindung zu keinem Feststoffausfall in der Extraktionsstufe kommt, ist somit eine kontinuierliche Arbeitsweise in jeder technischen Extraktionsapparatur zu realisieren. Dabei erlaubt die Ausführung der Extraktion in z. B. einer Gegenstromkolonne oder auch einer Mixer-Settler-Apparatur die Minimie-

rung der Extraktionsmittelmenge.

Im einzelnen ist das Verfahren der Erfindung nunmehr dadurch gekennzeichent, daß man der verunreinigten Katalysatorlösung in einer ersten Verfahrensstufe durch Extraktion mit Dialkylethern und Alkanolen mit jeweils 1-4 C-Atomen je Alkylrest die organischen Verunreinigungen sowie Essigsäure, Essigsäureanhydrid und Ethylidendiacetat entzieht und die Etherphase von der gereinigten promotorhaltigen Katalysatorlösung abtrennt ; daß man die Etherphase in einer zweiten Verfahrensstufe mit Jod und/oder Methyljodid behandelt, den ausgefallenen, promotorhaltigen Katalysatorkomplex abtrennt und in der gereinigten Katalysatorlösung der ersten Stufe auflöst ; daß man die Etherphase durch Destillation auftrennt, den zurückgewonnenen Dialkylether und das Alkanol erneut zur Extraktion einsetzt, aus dem zurückgewonnenen Gemisch aus Essigsäure, Essigsäureanhydrid und Ethylidendiacetat und der vereinigten gereinigten Katalysatorlösung unter Abdestillieren von restlichem Dialkylether und Alkanol erneut frische Katalysatorlösung bereitet und die als Rückstand der Etherphasendestillation verbleibenden organischen Verunreinigungen ausschleust.

Das Verfahren der Erfindung kann weiterhin bevorzugt und wahlweise dadurch gekennzeichnet sein, daß man

a) die Extraktion der verunreinigten Katalysatorlösung mit Dialkylether und Alkanol und die Behandlung der Etherphase mit Jod und/oder Methyljodid bei Temperaturen von 5-140 °C und Drücken von 1-30 bar durchführt ;

b) je Gewichtsteil verunreinigter Katalysatorlösung 0,5-20 Gewichtsteile Dialkylether und 0,03-0,4 Gewichtsteile Alkanol einsetzt ;

c) je Gewichtsteil Dialkylether 0,00005-0,01 Gewichtsteile Jod und/oder Methyljodid einsetzt ;

d) in der ersten Verfahrensstufe mindestens ein Alkanol der verunreinigten Katalysatorlösung und/oder dem Dialkylether zusetzt ;

e) aus der verunreinigten Katalysatorlösung zuerst die flüchtigen Anteile Essigsäure, Essigsäureanhydrid und Ethylidendiacetat abdestilliert, dann den Destillationsrückstand mit Dialkylether und Alkanol extrahiert und das gebildete Zweiphasengemisch in gereinigte, promotorhaltige, Katalysatorlösung und organische Verunreinigungen enthaltende Etherphase auftrennt.

Zur Herkunft der verunreinigten Katalysatorlösung ist zu sagen, daß die aus einem Carbonylierungsreaktor abfließende Reaktionsmischung destillativ in die gewünschten Endprodukte, besonders Essigsäureanhydrid, Essigsäure und/oder Ethylidendiacetat, und nichtumgesetzte, im Kreislauf geführte Ausgangsstoffe einerseits sowie die als Sumpfprodukt anfallende und im Kreislauf geführte Katalysatorlösung andererseits aufgetrennt wird. Ein Teilstrom dieser mit der Zeit durch undestillierbare organische Produkte verunreinigten Katalysatorlösung, welche je nach Verfahrensbedingungen bis zu 50 M-% an Essigsäureanhydrid, Essigsäure und/oder Ethylidendiacetat enthalten kann, wird aus dem Kreislauf der Katalysatorlösung entnommen und der Reinigung zugeführt. Die Katalysatorlösung enthält das Edelmetall Rhodium als Carbonylkomplex wie z. B.

$$[CH_3P(C_4H_9)_3]\ Rh(CO)I_4$$

oder

$$[CH_3P(C_4H_9)_3]\ Rh(CO)_2I_2\ .$$

Die Katalysatorlösung kann ferner als organische Promotoren bevorzugt einen oder mehrere der folgenden heterocyclischen aromatischen Stickstoffverbindungen oder Organophosphorverbindungen enthalten :

1. N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid ;
2. Tri-n-butyl-methyl-phosphoniumjodid, Trioctyl-methyl-phosphoniumjodid, Trilauryl-methylphosphoniumjodid, Triphenyl-methyl-phosphoniumjodid ;

Schließlich kann die Katalysatorlösung als anorganische Promotoren Alkalimetallsalze wie z. B. Lithiumjodid, Lithiumacetat, Kaliumjodid oder Natriumjodid und Verbindungen der carbonylbildenden Nichtedelmetalle Ce, Ti, Zr, Hf, Ge, Sn, Pb, V, Nb, Ta, As, Sb, Bi, Cr, Mo, W, Mn, Re, Fe, Co, Ni enthalten.

Die ausgeschleuste verunreinigte Katalysatorlösung wird mit dem Dialkylether und dem Alkanol bevorzugt bei 5-140 °C (1-30 bar) extrahiert. Hierbei werden die bei der Reaktion gebildeten undestillierbaren organischen Verunreinigungen sowie die in der Katalysatorlösung befindlichen Anteile Essigsäureanhydrid, Essigsäure und/oder Ethylidendiacetat extrahiert, während der Rh-Carbonylkomplex mit dem oder den Promotoren als flüssige Katalysatorphase zurückbleibt. Die abgezogene Etherphase wird sodann unter Zusatz von Jod und/oder Methyljodid bei bevorzugt 5-140 °C (1-30 bar) nachbehandelt, wobei ein rhodiumhaltiger Niederschlag ausfällt. Dieser Niederschlag wird abgetrennt und der gereinigten Katalysatorphase zugesetzt. Nach Abtrennen des Niederschlags aus der Etherphase wird Ether und Alkanol durch Destillation zurückgewonnen und in die Extraktion zurückgeführt. Anschließend werden Essigsäureanhydrid, Essigsäure und/oder Ethylidendiacetat redestilliert, wobei die undestillierbaren organischen Verunreinigungen als Rückstand anfallen. Die redestillierten Reaktionsprodukte werden der gereinigten Katalysatorphase (Rh-Carbonylkomplex und Promotorsalz) zugesetzt und nach destillativem Entfernen von gelösten Anteilen an Alkanol und Ether in den Carbonylierungsprozeß zurückgeführt. Die undestillierbaren organischen Verunreinigungen werden z. B. in einer Verbrennungsanlage vernichtet.

Das Verfahren der Erfindung kann in sowohl kontinuierlicher als auch diskontinuierlicher Betriebsweise durchgeführt werden.

Beispiel 1

Zur Entfernung der organischen Verunreinigungen werden dem Katalysatorkreislauf der Dimethylethercarbonylierung 1 000 g Katalysatorlösung der Zusammensetzung 7,35 M-% Rhodiumcarbonylkomplex $[CH_3P(C_4H_9)_3]$ $[Rh(CO)_2I_2]$ (≙ 12,0 g = 1,20 M-% Rh), 67,15 M-% Methyl-tri-n-butylphosphoniumjodid, 5,5 M-% organische Verunreinigungen und 20 M-% eines Gemisches aus Essigsäure, Essigsäureanhydrid und Ethylidendiacetat entnommen, mit 110 g Methanol versetzt und dann mit 2 000 g Diisopropylether (Sdp. 67,5 °C) ausgeschüttelt. Nach dem Entmischen der beiden flüssigen Phasen wird die Etherphase von der katalysatorhaltigen Phase abgetrennt, mit 0,8 g elementarem Jod versetzt und 2 h bei 70 °C unter Rückfluß gekocht. Dabei fällt ein Rh-haltiger Niederschlag aus. Dieser Niederschlag wird abfiltriert und in der katalysatorhaltigen Phase aufgelöst. Die filtrierte Etherphase wird sodann in 54 g Methanol, 1 920 g Diisopropylether, 120 g Essigsäure-Essigsäureanhydrid-Ethylidendiacetat-Gemisch und 50,8 g undestillierbare organische Verunreinigungen als teerartiger Rückstand (Rh-Gehalt 0,004 M-%) destillativ aufgetrennt. Das gewonnene Gemisch aus Essigsäure, Essigsäureanhydrid und Ethylidendiacetat wird der gereinigten Katalysatorphase zugegeben. Aus dem vereinigten Gemisch werden noch 56 g Methanol und 80 g Diisopropylether als Destillat entfernt, während 950 g gereinigte Katalysatorlösung mit einem Rh-Gehalt von 12 g verbleiben und dem Katalysatorkreislauf wieder zugesetzt werden. Die beiden Ether- und auch die Methanoldestillate werden vereinigt und zur erneuten Extraktion verwendet. Die Rückführungsrate des Rhodiums in den Carbonylierungsprozeß beträgt nach der Reinigung der abgezogenen verunreinigten Katalysatorlösung 99,98 %.

Beispiel 2

Zur Entfernung der organischen Verunreinigungen werden dem Katalysatorkreislauf der Methylacetatcarbonylierung 1 000 g Katalysatorlösung der Zusammensetzung 5,5 M-% Rhodiumcarbonylkomplex [Li] $[Rh(CO)_2I_2]$ (≙ 13,5 g = 1,35 M-% Rh), 69,2 M-% Lithiumjodid, 8,5 M-% organische Verunreinigungen und 16,8 M-% eines Gemisches aus Essigsäure, Essigsäureanhydrid und Ethylidendiacetat entnommen, mit 180 g Ethanol versetzt und dann mit 2 500 g Diisopropylether (Sdp. 67,5 °C) ausgeschüttelt. Nach dem Entmischen der beiden flüssigen Phasen wird die Etherphase von der katalysatorhaltigen Phase abgetrennt, mit 0,8 g elementarem Jod versetzt und 2 h bei 70 °C unter Rückfluß gekocht. Dabei fällt ein Rh-haltiger Niederschlag aus. Dieser Niederschlag wird abfiltriert und in der katalysatorhaltigen Phase aufgelöst. Die filtrierte Etherphase wird sodann in 2 370 g Diisopropylether, 83 g Ethanol, 95 g Essigsäure-Essigisäureanhydrid-Ethylidendiacetat-Gemisch und 77,7 g undestillierbare organische Verunreinigungen als teerartiger Rückstand (Rh-Gehalt 0,004 M-%) destillativ aufgetrennt. Das gewonnene Gemisch aus Essigsäure, Essigsäureanhydrid und Ethylidendiacetat wird der gereinigten Katalysatorphase zugegeben. Aus dem vereinigten Gemisch werden noch 130 g Diisopropylether und 97 g Ethanol als Destillat entfernt, während 923 g gereinigte Katalysatorlösung mit einem Rh-Gehalt von 13,5 g verbleiben und dem Katalysatorkreislauf wieder zugesetzt werden. Die beiden Ether- und auch die Ethanoldestillate werden vereinigt und zur erneuten Extraktion verwendet. Die Rückführungsrate des Rhodiums in den Carbonylierungsprozeß beträgt nach der Reinigung der abgezogenen verunreinigten Katalysatorlösung 99,98 %.

Beispiel 3

Zur Entfernung der organischen Verunreinigungen werdem dem Katalysatorkreislauf der Methylacetatcarbonylierung 1 000 g Katakysatorlösung der Zusammensetzung 6,2 M-% Rhodiumcarbonylkomplex $C_5H_9N_2[Rh(CO)_2I_2]$ (≙ 12,5 g = 1,25 M-% Rh), 64,0 M-% N,N-Dimethylimidazoliumjodid ($C_5H_9N_2I$), 7,3 M-% organische Verunreinigungen und 22,5 M-% eines Gemisches aus Essigsäure, Essigsäureanhydrid und Ethylidendiacetat entnommen. Nach Abdestillieren der flüchtigen Komponenten Essigsäure, Essigsäureanhydrid und Ethylidendiacetat wird der als Rückstand verbleibende verunreinigte, promotorhaltige Katalysatorkomplex mit einem Gemisch aus 200 g i-Propanol und 2 500 g Diethylether (Sdp. 34,6 °C) aufgelöst, wobei sich zwei flüssige Phasen bilden, und danach ausgeschüttelt. Nach dem Entmischen der beiden flüssigen Phasen wird die Etherphase von der katalysatorhaltigen Phase abgetrennt, mit 0,8 g elementarem Jod versetzt und 2 h bei 36 °C unter Rückfluß gekocht. Dabei fällt ein Rh-haltiger Niederschlag aus. Dieser Niederschlag wird abfiltriert und in der katalysatorhaltigen Phase aufgelöst. Die filtrierte Etherphase wird sodann in 2 397 g Diethylether, 105 g i-Propanol und 67,6 g undestillierbare organische Verunreinigungen als teerartiger Rückstand (Rh-Gehalt 0,003 M-%) destillativ aufgetrennt. Das zuvor abdestillierte Gemisch aus Essigsäure, Essigsäureanhydrid und Ethylidendiacetat wird der gereinigten Katalysatorphase zugegeben. Aus dem vereinigten Gemisch werden noch 103 g Diethylether und 95 g i-Propanol als Destillat entfernt, während 933 g gereinigte Katalysatorlösung mit einem Rh-Gehalt von 12,5 g verbleiben und dem Katalysatorkreislauf wieder zugesetzt werden. Die beiden Ether- und auch die i-Propanoldestillate werden vereinigt und zur erneuten Extraktion verwendet. Die Rückführungsrate des Rhodiums in den Carbonylierungsprozeß beträgt nach der Reinigung der abgezogenen verunreinigten Katalysatorlösung 99,98 %.

Beispiel 4

Zur Entfernung der organischen Verunreinigungen werden dem Katalysatorkreislauf der Dimethylethercarbonylierung kontinuierlich 50 g/h Katalysatorlösung der Zusammensetzung

6,7 M-% Rhodiumcarbonylkomplex $[CH_3P(C_4H_9)_3][Rh(CO)_2I_2]$ (≙ 0,55 g = 1,1 M-% Rh), 64,5 M-% Methyl-tri-n-butylphosphoniumjodid, 4,8 M-% organische Verunreinigungen und 24,0 M-% eines Gemisches aus Essigsäure, Essigsäureanhydrid und Ethylidendiacetat entnommen, mit 5 g/h Methanol versetzt und dann in einer 3stufigen Mixer-Settler-Apparatur mit 77 g/h eines Gemisches der Zusammensetzung 5 g Methanol und 72 g Diisopropylether im Gegenstrom in Berührung gebracht. Die aus dem Trenngefäß ablaufende Etherphase wird mit 0,04 g/h elementarem Jod versetzt und 2 h bei 70 °C unter Rückfluß gekocht. Dabei fällt ein Rh-haltiger Niederschlag aus. Dieser Niederschlag wird abfiltriert und in der ebenfalls aus dem Trenngefäß ablaufenden katalysatorhaltigen Phase aufgelöst. Die filtrierte Etherphase wird sodann in 4,5 g/h Methanol, 67 g/h Diisopropylether, 10 g/h Essigsäure-Essigsäureanhydrid-Ethylidendiacetat-Gemisch und 2,3 g/h undestillierbare organische Verunreinigungen als teerartiger Rückstand (Rh-Gehalt 0,004 M-%) destillativ aufgetrennt. Das gewonnene Gemisch aus Essigsäure, Essigsäureanhydrid und Ethylidendiacetat wird der gereinigten Katalysatorphase zugegeben. Aus dem vereinigten Gemisch werden noch 5,5 g/h Methanol und 5 g/h Diisopropylether als Destillat entfernt, während 47,7 g/h gereinigte Katalysatorlösung mit einem Rh-Gehalt von 0,55 g verbleiben und dem Katalysatorkreislauf wieder zugesetzt werden. Die beiden Ether- und auch die Methanoldestillate werden vereinigt und zur erneuten Extraktion verwendet. Die Rückführungsrate des Rhodiums in den Carbonylierungsprozeß beträgt nach der Reinigung der abgezogenen verunreinigten Katalysatorlösung 99,98 %.

**Patentansprüche**

1. Verfahren zur Reinigung und Rückgewinnung der bei der Carbonylierung von Methylacetat und/oder Dimethylether anfallenden verunreinigten Katalysatorlösung, welche Carbonylkomplexe des Rhodiums, quaternäre heterocyclische aromatische Stickstoffverbindungen oder quaternäre Organophosphorverbindungen als organische Promotoren, und/oder Alkalimetallsalze und gegebenenfalls Verbindungen carbonylbildender Nichtedelmetalle als anorganische Promotoren, undestillierbare organische Verunreinigungen sowie Essigsäure, Essigsäureanhydrid und Ethylidendiacetat enthält, dadurch gekennzeichnet, daß man der verunreinigten Katalysatorlösung in einer ersten Verfahrensstufe durch Extraktion mit Dialkylethern und Alkanolen mit jeweils 1-4 C-Atomen je Alkylrest die organischen Verunreinigungen sowie Essigsäure, Essigsäureanhydrid und Ethylidendiacetat entzieht und die Etherphase von der gereinigten promotorhaltigen Katalysatorlösung abtrennt ; daß man die Etherphase in einer zweiten Verfahrensstufe mit Jod und/oder Methyljodid behandelt, den ausgefallenen, promotorhaltigen Katalysatorkomplex abtrennt und in der gereinigten Katalysatorlösung der ersten Stufe auflöst ; daß man die Etherphase durch Destillation auftrennt, den zurückgewonnenen Dialkylether und das Alkanol erneut zur Extraktion einsetzt, aus dem zurückgewonnenen Gemisch aus Essigsäure, Essigsäureanhydrid und Ethylidendiacetat und der vereinigten gereinigten Katalysatorlösung unter Abdestillieren von restlichem Dialkylether und Alkanol erneut frische Katalysatorlösung bereitet und die als Rückstand der Etherphasendestillation verbleibenden organischen Verunreinigungen ausschleust.

2. Verfahren nach Anspruch 1, dadurch gekennzeichnet, daß man die Extraktion der verunreinigten Katalysatorlösung mit Dialkylether und Alkanol und die Behandlung der Etherphase mit Jod und/oder Methyljodid bei Temperaturen von 5-140 °C und Drücken von 1-30 bar durchführt.

3. Verfahren nach mindestens einem der Ansprüche 1 und 2, dadurch gekennzeichnet, daß man je Gewichtsteil verunreinigter Katalysatorlösung 0,5-20 Gewichtsteile Dialkylether und 0,03-0,4 Gewichtsteile Alkanol einsetzt.

4. Verfahren nach mindestens einem der vorhergehenden Ansprüche, dadurch gekennzeichnet, daß man je Gewichtsteil Dialkylether 0,00005-0,01 Gewichtsteile Jod und/oder Methyljodid einsetzt.

5. Verfahren nach mindestens einem der vorhergehenden Ansprüche, dadurch gekennzeichnet, daß man in der ersten Verfahrensstufe mindestens ein Alkanol der verunreinigten Katalysatorlösung und/oder dem Dialkylether zusetzt.

6. Verfahren nach mindestens einem der vorhergehenden Ansprüche, dadurch gekennzeichnet, daß man aus der verunreinigten Katalysatorlösung zuerst die flüchtigen Anteile Essigsäure, Essigsäureanhydric und Ethylidendiacetat abdestilliert, dann den Destillationsrückstand mit Dialkylether und Alkanol extrahiert und das gebildete Zweiphasengemisch in gereinigte, promotorhaltige Katalysatorlösung und organische Verunreinigungen enthaltende Etherphase auftrennt.

**Claims**

1. A process for purifying and recovery processing the contaminated catalyst solution obtained in the carbonylation of methyl acetate and/or dimethyl ether which contains carbonyl complexes of rhodium, quaternary heterocyclic aromatic nitrogen compounds or quaternary organophosphorus compounds as organic promotors, and/or alkali metal salts and possibly compounds of carbonyl-forming non-noble metals as inorganic promotors, undistillable organic impurities and also acetic acid, acetic anhydride and ethylidene diacetate, which comprises stripping the contaminated catalyst solution in a first process stage by extraction with dialkyl ethers and alkanols each of 1-4 carbon atoms per alkyl radical of the organic impurities and also acetic acid, acetic anhydride and ethylidene diacetate and separating the ether phase from the purified promotor-containing catalyst solution, treating the ether phase in a second process stage with iodine and/or methyl iodide, separating off the precipitated promotor-containing catalyst complex and dissolving it in the purified catalyst solution of the first stage, separating the ether phase by distillation, using the recovered dialkyl ether and the alkanol again for extraction, using the recovered mixture of acetic acid, acetic anhydride and ethylidene diacetate and the combined purified catalyst solution to prepare a fresh catalyst solution again by distilling off the residual dialkyl ether and alkanol, and channeling out the organic impurities remaining behind as residue of the ether phase distillation.

2. The process as claimed in claim 1, wherein the extraction of the contaminated catalyst solution with dialkyl ether and alkanol and the treatment of the ether phase with iodine and/or methyl iodide are carried out at temperatures of 5-140 °C and pressures of 1-30 bar.

3. The process as claimed in at least one of claims 1 and 2, wherein 0.5-20 parts by weight of dialkyl ether and 0.03-0.4 part by weight of alkanol are used per part by weight of contaminated catalyst solution.

4. The process as claimed in at least one of the preceding claims, wherein 0.00005-0.01 part by weight of iodine and/or methyl iodide is used per part by weight of dialkyl ether.

5. The process as claimed in at least one of the preceding claims, wherein in the first process stage at least one alkanol is added to the contaminated catalyst solution and/or the dialkyl ether.

6. The process as claimed in at least one of the preceding claims, wherein first the volatile constituents acetic acid, acetic anhydride and ethylidene diacetate are distilled out of the contaminated catalyst solution, then the distillation residue is extracted with dialkyl ether and alkanol and the two-phase mixture formed is separated into purified, promotor-containing catalyst solution and the ether phase which contains organic impurities.

**Revendications**

1. Procédé pour la purification et la récupération de la solution de catalyseur contaminée se formant dans la carbonylation de l'acétate de méthyle et/ou de l'oxyde de diméthyle, qui contient des complexes carbonyles du rhodium, des composés azotés aromatiques hétérocycliques quaternaires ou des composés organophosphorés quaternaires comme promoteurs organiques et/ou des sels de métaux alcalins et éventuellement des composés de métaux non nobles formateurs de carbonyles comme promoteurs inorganiques, des impuretés organiques non distillables ainsi que de l'acide acétique, de l'anhydride acétique et du diacétate d'éthylidène, caractérisé en ce que l'on retire, dans un premier stade opératoire, de la solution de catalyseur contaminée les impuretés organiques ainsi que l'acide acétique, l'anhydride acétique et le diacétate d'éthylidène par extraction par des oxydes de dialkyles et des alcanols ayant chaque fois 1-4 atomes de carbone dans chaque reste alkyle et l'on sépare la phase éthérée de la solution de catalyseur contenant les promoteurs, purifiée ; en ce que l'on traite la phase éthérée, dans un second stade opératoire, par l'iode et/ou l'iodure de méthyle, on sépare le complexe de catalyseur contenant les promoteurs, précipité et on le dissout dans la solution de catalyseur purifiée du premier stade opératoire ; en ce que l'on sépare la phase éthérée par distillation, on utilise à nouveau pour l'extraction l'oxyde de dialkyle et l'alcanol récupérés, on prépare à nouveau une solution de catalyseur fraîche à partir du mélange récupéré d'acide acétique, d'anhydride acétique et de diacétate d'éthylidène et de la solution de catalyseur purifiée réunie avec élimination par distillation de l'oxyde de dialkyle et de l'alcanol résiduels et on écluse au dehors les impuretés organiques restant comme résidu de la distillation de la phase éthérée.

2. Procédé selon la revendication 1, caractérisé en ce que l'on met en œuvre l'extraction de la solution de catalyseur contaminée au moyen d'oxyde de dialkyle et d'alcanol et le traitement de la phase éthérée par l'iode et/ou l'iodure de méthyle à des températures de 5-140 °C et sous des pressions de 1-30 bars.

3. Procédé selon l'une au moins des revendications 1 et 2, caractérisé en ce que l'on utilise 0,5-20 parties en poids d'oxyde de dialkyle et 0,03-0,4 partie en poids d'alcanol par partie en poids de solution de catalyseur contaminée.

4. Procédé selon l'une au moins des revendications précédentes, caractérisé en ce que l'on utilise 0,00005-0,01 partie en poids d'iode et/ou d'iodure de méthyle par partie en poids d'oxyde de dialkyle.

5. Procédé selon l'une au moins des revendications précédentes, caractérisé en ce que l'on ajoute dans le premier stade opératoire au moins un alcanol à la solution de catalyseur contaminée et/ou à l'oxyde de dialkyle.

6. Procédé selon l'une au moins des revendications précédentes, caractérisé en ce que l'on sépare d'abord par distillation les fractions volatiles acide acétique, anhydride acétique et diacétate d'éthylidène de la solution de catalyseur contaminée, on extrait ensuite le résidu de distillation par l'oxyde de dialkyle et l'alcanol et on sépare le mélange à deux phases formé en une solution de catalyseur contenant les promoteurs, purifiée et une phase éthérée contenant les impuretés organiques.